# EUROPEAN PATENT APPLICATION

(11) **EP 4 646 988 A1**
(43) Date of publication of application: **12.11.2025**
(21) Application number: 23914114.6
(22) Date of filing: 17.01.2023
(51) Int. Cl.: A47L 11/40

(54) **SURFACE CLEANING SYSTEM, AND WASHING, DRYING AND STERILIZATION METHOD APPLIED TO BRUSH ROLLER**

(30) Priority: 06.01.2023 CN 202310016885
(71) Applicant: Suzhou Eup Electric Co. Ltd., Suzhou, Jiangsu 215011 (CN)
(72) Inventor: HUANG, Yuyuan, Suzhou, Jiangsu 215011 (CN)
(74) Representative: ABG Intellectual Property Law, S.L.
(86) International application number: PCT/CN2023/072501
(87) International publication number: WO 2024/145958

(57) **Abstract**

A surface cleaning system, and a method for cleaning, drying, and sterilizing a brush roller (14). The system comprises a surface cleaning device (10) and a base station (20). The base station (20) comprises at least one heating wall structure (8) having a controllable heating element (82), and a power supply unit (7). The at least one heating wall structure (8) is configured to circumferentially cover at least part of the periphery of an exposed portion (141) of the brush roller (14) when the surface cleaning device (10) is docked at the base station (20). Via efficient heating capability of the at least one heating wall structure (8) in the base station (20), and the ability of a suction motor (42) in the surface cleaning device (10) to drive a fluid flow, a drying process for the brush roller (14) can be completed within a short time.

## Description

This application claims priority to Chinese patent application No. 202310016885.3, filed on January 6, 2023, with the title of "Surface Cleaning System". The entire contents of which are incorporated by reference into this application.

### FIELD OF THE INVENTION

The present application relates to the field of cleaning equipment, in particular to a surface cleaning system and a method for cleaning, drying and sterilizing a brush roller.

### BACKGROUND

Surface cleaning machines that use cleaning liquids, such as clean water, to perform cleaning operations, have been widely used in daily life. For example, wet floor cleaning devices that uses water or a water/cleaning agent mixture to scrub the floor are equipped with brush rollers that contact the floor and carry a recovery container for recovering used cleaning liquid. The brush rollers can pick up used cleaning liquid that has become dirty liquid along with the debris on the floor and transport these to the recovery container under the action of a suction motor.

During the operation of this type of surface cleaning machine, the brush roller is constantly in contact with dirty liquid. The brush roller is thus required to be cleaned after the operation, and must be actively dried after cleaning to prevent odor and mold growth.

In the prior art, there are common problems in the process of used brush rollers, such as incomplete cleaning, failure of effective sterilization, and a long drying time.

### SUMMARY

The present application provides a surface cleaning system and a method for cleaning, drying, and sterilizing a brush roller, which are used to treat a used surface cleaning device.

In a first aspect, a surface cleaning system is provided, comprising:
a surface cleaning device, comprising a suction nozzle, a brush roller, a brush roller motor for driving the brush roller to rotate, a dirty liquid recovery tank and a suction motor, wherein the brush roller comprises a roller body and a cleaning element which covers an outer peripheral surface of the roller body and is capable of being wetted by liquid, and the brush roller has an exposed portion exposed outward;
a base station, configured to allow the surface cleaning device to dock, wherein the base station comprises at least one heating wall structure having a controllable heating element, and the at least one heating wall structure is configured to circumferentially cover at least a portion of an outer periphery of the exposed portion while the surface cleaning device is docked at the base station; and
a control unit, wherein the control unit is at least partially arranged at the surface cleaning device, and the control unit is configured to perform a self-cleaning process of cleaning the brush roller with water, and a drying process of drying the brush roller while the surface cleaning device is docked at the base station; wherein during execution of the drying process, the control unit is configured to control the suction motor and the brush roller motor respectively to be in continuous operation and to control the heating element to generate heat for at least a period of time.

In this surface cleaning system, with the help of efficient heating capability of at least one heating wall structure and the ability of the suction motor to drive the fluid flow, the drying process for the brush roller can be completed in a short time, which greatly reduces the time the user spends watching over the system and improves the convenience of using the surface cleaning device.

In a possible embodiment, the at least one heating wall structure has an upper surface facing the brush roller, and the heating element is configured so that a temperature of the upper surface is greater than or equal to 50°C during the drying process and when the heating element is in a heating period.

In a possible implementation manner, the upper surface is arc-shaped.

In a possible embodiment, the at least one heating wall structure is configured to contact the exposed portion while the surface cleaning device is docked at the base station.

In a possible implementation, the base station comprises a recess capable of receiving at least a portion of the brush roller while the surface cleaning device is docked at the base station, and the at least one heating wall structure comprises at least a portion of a wall of the recess.

In a possible implementation, the recess has a front wall and a bottom wall, and the at least one heating wall structure comprises at least a portion of the front wall and at least a portion of the bottom wall.

In a possible implementation, the at least one heating wall structure comprises a heat-conducting part, and the heating element is embedded in the heat-conducting part.

In a possible implementation manner, the at least one heating wall structure circumferentially covers more than fifty percent of a surface area of the exposed portion while the surface cleaning device is docked at the base station.

In a possible implementation, the self-cleaning process is configured to be performed before the drying process, and the control unit is configured to control the heating element to generate heat during the self-cleaning process.

In a possible embodiment, the surface cleaning device also comprises: a water tank, a liquid distributor facing the brush roller, and a pump for conveying liquid from the water tank to the liquid distributor; wherein the water used during the self-cleaning process comes from the water tank.

In a possible implementation, the control unit comprises a first controller located at the surface cleaning device, and a second controller located at the base station, and the first controller is in communication with the second controller while the surface cleaning device is docked at the base station.

In a possible embodiment, the control unit is configured to perform a sterilizing process of the brush roller using steam while the surface cleaning device is docked at the base station, and the sterilizing process is configured to be performed between the self-cleaning process and the drying process; wherein the control unit is configured to control the heating element to generate heat to heat water in the recess and/or the cleaning element soaked in water to generate the steam during the sterilizing process, and the control unit is configured to control the brush roller motor to drive the brush roller to rotate during the sterilizing process.

In a possible implementation, during the sterilizing process, the control unit controls the brush roller motor to drive the brush roller to rotate at a speed lower than a rotation speed of the brush roller when the surface cleaning device performs a surface cleaning operation.

In a possible implementation, during the drying process, the control unit controls the heating element to stop heating in advance before the drying process ends.

In a second aspect, a method for drying a brush roller is provided, which is applied to a surface cleaning device, and the method comprises: docking the surface cleaning device to a base station provided with at least one heating wall structure, wherein the at least one heating wall structure comprises a controllable heating element and the at least one heating wall structure is configured to circumferentially cover at least a portion of a periphery of the brush roller during a period when the surface cleaning device is docked at the base station; and controlling the heating element to generate heat so as to heat the brush roller via the at least one heating wall structure, and starting a brush roller motor of the surface cleaning device and a suction motor of the surface cleaning device to both participate in operation.

In this method, on one hand, the brush roller is heated by at least one heating wall structure, and on the other hand, with the help of the air flow effect generated by the suction motor, the moisture on the brush roller can be quickly evaporated and removed in real time, thereby achieving the purpose of quickly drying the brush roller.

In a possible implementation, the at least one heating wall structure contacts the brush roller while enclosing at least a portion of a periphery of the brush roller.

In a possible implementation manner, the method further comprises: controlling the heating element to stop heating in advance before shutting down the brush roller motor and the suction motor.

In a third aspect, a method for cleaning a brush roller is provided, which is applied to a surface cleaning device, the method comprising: docking the surface cleaning device to a base station provided with at least one heating wall structure to clean the brush roller; wherein the at least one heating wall structure comprises a controllable heating element and the at least one heating wall structure is configured to circumferentially cover at least a portion of a periphery of the brush roller while the surface cleaning device is docked at the base station; and controlling the heating element to generate heat so that the brush roller is cleaned in a heated environment.

In this method, the brush roller and/or the water flowing into the recess is heated by means of at least one heating wall structure, so that the brush roller can be cleaned in a heated environment. It not only has a good cleaning effect, but also simultaneously achieves high-temperature sterilization of the brush roller, so that users can use the system with greater peace of mind.

In a possible implementation, the method comprises: applying water required for cleaning the brush roller to the brush roller.

In a possible implementation, the method comprises: transferring used water that has been used for cleaning the brush roller from the brush roller.

In a possible embodiment, the surface cleaning device is also provided with a suction nozzle, a dirty liquid recovery tank and a suction motor, and the method comprises: transferring the used water that has been used for cleaning the brush roller from the brush roller to the dirty liquid recovery tank utilizing the suction motor.

In a possible implementation, the heating wall structure contacts the brush roller while circumferentially covering at least a portion of the periphery of the brush roller.

In a fourth aspect, a method for sterilizing a brush roller is provided, which is applied to a surface cleaning device, and the method comprises: docking the surface cleaning device to a base station provided with at least one heating wall structure, wherein the at least one heating wall structure comprises a controllable heating element and the at least one heating wall structure is configured to circumferentially cover at least a portion of a periphery of the brush roller during a period when the surface cleaning device is docked at the base station; controlling the heating element to generate heat so as to convert water located at the at least one heating wall structure and/or water absorbed by the brush roller into steam; and controlling a brush roller motor of the surface cleaning device to drive the brush roller to rotate in an steam-filled environment.

In the method, water in the recess and/or water absorbed in the cleaning element is heated by at least one heating wall structure to generate steam, and the brush roller can be sterilized by the steam, so that the user can later use the surface cleaning device carrying the brush roller with more peace of mind.

In a possible implementation, the method further comprises: controlling the brush roller motor to drive the brush roller to rotate in a steam-filled environment at a rotation speed lower than the rotation speed of the brush roller when the surface cleaning device performs a surface cleaning operation.

### BRIEF DESCRIPTION OF THE DRAWINGS

FIG.1 is a three-dimensional structural diagram of a surface cleaning system provided by the present application, wherein a surface cleaning device is docked at a base station;
FIG.2 is a perspective view of a surface cleaning device provided by the present application;
FIG.3 is a front cross-sectional view of a surface cleaning device provided by the present application, wherein the dirty liquid recovery tank is removed and the handle is omitted;
FIG.4 is a three-dimensional diagram of a base station provided in an embodiment of the present application;
FIG.5 is an exploded view of a base station provided in an embodiment of the present application;
FIG.6 is a diagram showing the cleaning base docked to the base station when the surface cleaning device is docked at the base station, according to an embodiment of the present application;
FIG.7 is a control logic diagram of a surface cleaning system provided in an embodiment of the present application;
FIG.8 is a sequence diagram of various processes provided in an embodiment of the present application;
FIG.9 is a diagram showing the stages of a self-cleaning process provided in an embodiment of the present application.

### DESCRIPTION OF PREFERRED EMBODIMENTS

To describe technical contents, structural features, achieved purpose and effect of the present application in detail, technical solutions in embodiments of the present application will be described below in conjunction with the drawings. Obviously, the described embodiments are only a part of embodiments of the present application, rather than all embodiments. In the following description, for the purpose of explanation, some specific details are presented to provide a detailed description of various exemplary embodiments or embodiments of the invention. However, various exemplary embodiments may also be implemented without these specific details or with one or more equivalent arrangements. In addition, various exemplary embodiments may be different, but not necessarily exclusive. For embodiment, without departing from the concept of the present application, specific shapes, structures and characteristics of an exemplary embodiment may be used or implemented in another exemplary embodiment.

For the purpose of description in relation to the drawings, the terms "upper", "lower", "front", "rear", "inner", "outer" and their derivatives are used in a defined positional relationship relative to the direction in which the user stands on the surface to be cleaned and pushes the surface cleaning device 10 (i.e., the pushing direction from the back to the front). As used herein, the term "rear side" refers to a position behind at least one other component, but does not necessarily mean behind all other components. However, it should be understood that the present invention can take various alternative orientations unless the opposite orientation is explicitly indicated.

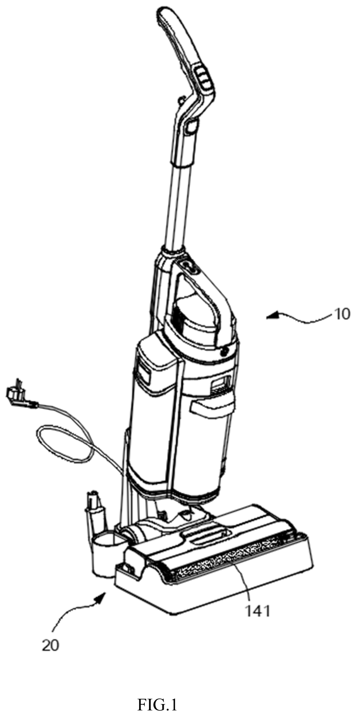

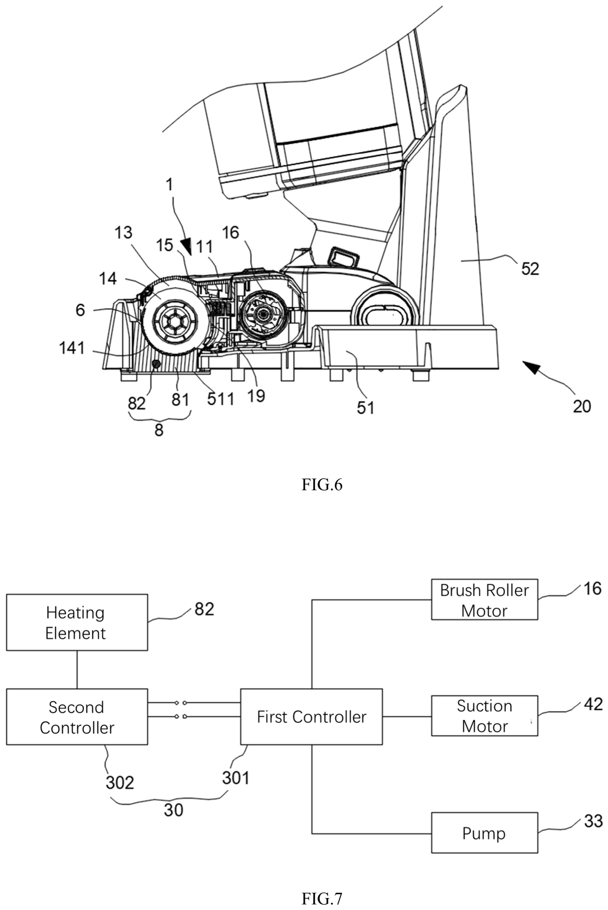

FIG.1 shows a surface cleaning system provided by the present invention, which comprises a surface cleaning device 10, a base station 20 capable of docking with the surface cleaning device 10, and a control unit 30 (see FIG.7). In the surface cleaning system, the surface cleaning device 10 can be detached from the base station 20 to independently perform operations of cleaning a surface to be cleaned. The surface cleaning device 10 can also be placed on the base station 20 to undergo self-cleaning process, sterilizing process and drying process, which are mainly for components such as brush rollers in the surface cleaning device.

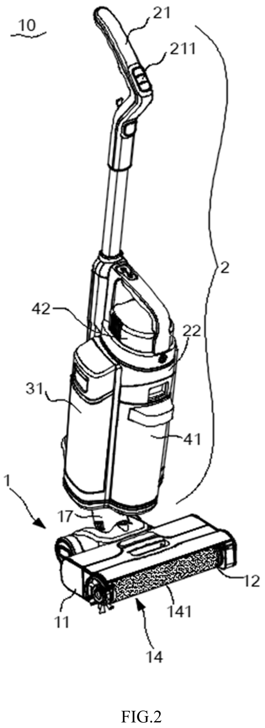

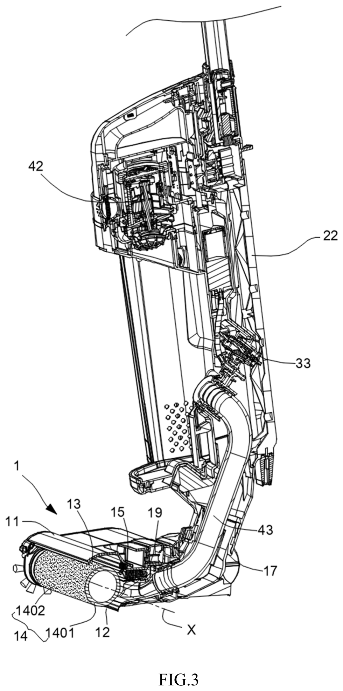

As shown in FIGS. 2-3, the surface cleaning device 10 is an upright cleaning device, which comprises a cleaning base 1 and a handle body portion 2 located on the upper side of the cleaning base 1. The lower portion of the handle body portion 2 is pivotally connected to the cleaning base 1, and the handle body portion 2 has a storage position in which it is upright relative to the cleaning base 1 for storage and an operation position in which it is tilted backward relative to the cleaning base 1 for the convenience of the user to push the surface cleaning device 10.

The cleaning base 1 comprises a base body 11 constituting its own outer contour, a roller chamber 13 located at the front of the base body 11, a suction nozzle 19 located inside the cleaning base 1 and adjacent to the rear side of the roller chamber 13, a brush roller 14 rotatably arranged at the roller chamber 13, and a liquid distributor 15 partially extending into the roller chamber 13. A rotation joint 17 is provided on the base body 11, and the lower part of the handle body portion 2 is fixedly connected to the rotation joint 17, thereby enabling the handle body portion 2 to rotate back and forth between the upright storage position and the backward tilted operation position.

The liquid distributor 15 faces the brush roller 14 and is configured to distribute water from a water delivery path (not shown) onto the brush roller 14.

The brush roller 14 comprises a cylindrical roller body 1401 defining a rotation axis X extending in a left-right direction and a cleaning element 1402 covering the outer peripheral surface of the roller body 1401, and the cleaning element 1402 is configured to be wetted by a liquid such as water. In this embodiment, the cleaning element 1402 is fibrous fluff. In other embodiments, the cleaning element may also be other types such as sponges and fabrics. The cleaning base 1 is further provided with a brush roller motor (not shown in the figure) connected to the brush roller 14 to drive the brush roller 14 to rotate around the rotation axis X.

In this embodiment, the front and lower part of the roller chamber 13 have a through opening 12, and part of the brush roller 14 is exposed from the opening 12 to constitute an exposed portion 141 of the brush roller 14, which is exposed to the outside to be able to contact the surface to be cleaned.

In this embodiment, the front and lower part of the roller chamber 13 have the opening 12, and a portion of the brush roller 14 is exposed from the opening 12 to form an exposed portion 141 of the brush roller 14, which can contact the surface to be cleaned.

The handle body portion 2 comprises a handle 21 arranged at the upper part and a body 22. The handle 21 is fixedly connected to the top of the body 22 and is adapted for the user to hold. The user can use the handle 21 to hold the surface cleaning device 10 with one hand and use it to push the cleaning base 1 of the surface cleaning device 10 to move back and forth on the surface to be cleaned in the backward tilted operation position. The handle 21 is also provided with an operation element 211 that is connected to the control unit 30 for the user to control the operation of the surface cleaning device 10. The specific form of the operation element 211 is not limited to a button, a trigger, an actuator, a switch, etc. In other embodiments, the operation element may also be arranged in other places of the surface cleaning device, such as on the top wall or on a side wall of the handle body portion at the lower part of the handle. The operation element may also be a portable remote controller or may be arranged on a handheld terminal that can interact with the surface cleaning device.

The body 22 can receive and carry multiple working parts of the handle body portion 2. These multiple working parts comprise a water tank 31 that can store and provide clean water outward. Wherein the water tank 31 is preferably detachably mounted on the body 22, so that the user can add clean water thereinto.

The water tank 31 is in fluid communication with the liquid distributor 15 on the cleaning base 1 and forms a water supply path (not shown in the figure) for water flow. A selectively activated pump 33 is also provided on the water supply path. By controlling the pump 33 to be turned on, liquid can be selectively delivered to the liquid distributor 15, and by controlling the pump 33 to be turned off, the fluid communication between the water tank 31 and the liquid distributor 15 can be blocked. In this embodiment, the pump 33 is arranged inside the body 22. In other embodiments, the pump can also be arranged inside the base body 11 of the cleaning base 1. The water supply unit including the water tank 31, the water supply path, the pump 33 and the liquid distributor 15 is configured to supply water to the brush roller 14 when required.

The working parts arranged on the body 22 also comprise a dirty liquid recovery tank 41 that can receive and store dirty liquid, and a suction motor 42 arranged on the upper side of the dirty liquid recovery tank 41. The suction nozzle 19 in the cleaning base 1, the dirty liquid recovery tank 41 and the suction motor 42 are fluidically connected in sequence, thus a recovery flow path 43 is provided between the suction nozzle 19 and the dirty liquid recovery tank 41. The suction motor 42 is configured to be able to drive the fluid containing air from the suction nozzle 19 toward the dirty liquid recovery tank 41 along the recovery flow path 43. The dirty liquid recovery tank 41 is detachably mounted on the body 22 to facilitate the user to dump the dirty liquid.

When the surface cleaning device 10 is in operation, the pump 33 is started, the water in the water tank 31 reaches the liquid distributor 15 via the water delivery path and is distributed to the brush roller 14 by the liquid distributor 15, and the rotating brush roller 14 uses the water to scrub the surface to be cleaned that it contacts. At the same time, the suction motor 42 is started and an air flow is generated, and the brush roller 14 carries dirty liquid and debris picked up from the surface to be cleaned into the recovery flow path 43 along with the air flow, and the dirty liquid and debris will finally be stored in the dirty liquid recovery tank 41.

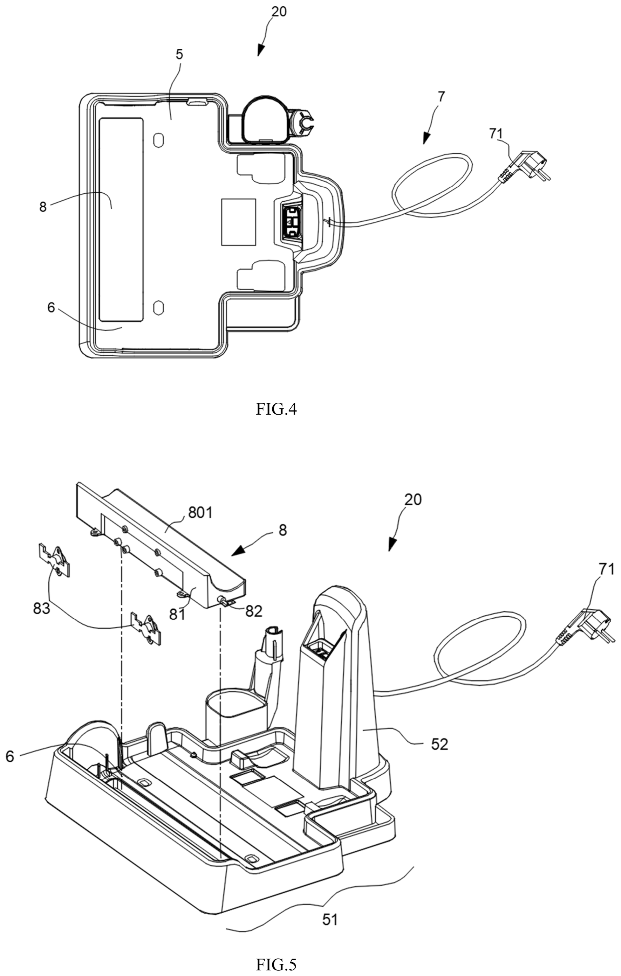

In FIGS. 4-5, the base station 20 of the surface cleaning system comprises a housing 5, a recess 6 provided on the upper part of the housing 5 and capable of receiving the brush roller 14 when the surface cleaning device 10 is docked at the base station 10, and a power supply unit 7. The power supply unit 7 comprises an electric plug 71 that can be electrically connected to a household socket. In the base station 20, a charging component (not shown in the figure) for charging the surface cleaning device 10 can also be arranged, and the charging component is electrically connected to the power supply unit.

The housing 5 constitutes the outer contour of the base station 20, and comprises a tray portion 51 for supporting the surface cleaning device 10 and a boss portion 52 located at the rear side of the tray portion 51 and protruding upward relative to the tray portion 51. The tray portion 51 is configured to dock with the bottom portion of the cleaning base 1, and the recess 6 is located at the front portion of the tray portion 51 and is recessed downward. The boss portion 52 is generally docked with the handle body portion 2.

The base station 20 further comprises a heating wall structure 8, which comprises a portion of the front wall of the recess and a portion of the bottom wall of the recess 6. The heating wall structure 8 is configured to radiate heat outward. In other embodiments, multiple heating wall structures 8 might be provided.

The heating wall structure 8 comprises a heat-conducting part 81 and a heating element 82 embedded in the heat-conducting part 81. In other embodiments, the heating element 82 may also be located outside the heat-conducting part 81, as long as it is provided with a heat-conducting connection with the heat-conducting part 81. The heating element 82 can be arranged in various shapes, such as a disc, a strip, a multi-point arrangement, a mesh, etc. The material of the heat-conducting part 81 can be a material with excellent thermal conductivity such as aluminum. The heating element 82 is electrically connected to the power supply unit 7 and the power supply unit 7 provides the power required for heating.

To ensure that the heating is controlled, a temperature controller 83 is further provided at the heating wall structure 8, and the temperature controller 83 is in communication with the control unit 30. The control unit 30 is configured to control the heating wall structure 8 to generate heat as required with the help of the temperature controller 83.

In this embodiment, the heating wall structure 8 has an arc-shaped upper surface 801. The upper surface 801 of the heating wall structure 8 directly contacts the cleaning element 1402 at the exposed portion 141 when the surface cleaning device 10 is docked at the base station 20. The temperature of the upper surface 801 during operation is determined by the heating power of the heating element 82. In one embodiment of the present application, the heating element 82 is configured so that the temperature of the upper surface 801 is greater than or equal to 50°C during a drying process and when the heating element 82 is in a heating period. In some preferred embodiments, the heating power of the heating element 82 is configured to achieve that the temperature of the upper surface 801 is greater than or equal to 72°C. To make the heating wall structure 8 have a better heating capacity, the temperature of the upper surface 801 can be configured to be greater than or equal to 100°C.

In some embodiments, a protruding dot array (not shown) or other similar structures may be arranged on the upper surface 801 of the heating wall structure 8. The protruding dot array can extend into the interior of the brush roller 14 when the surface cleaning device 10 is docked at the base station 20. Such a protruding dot array can transfer heat to the brush roller 14, and comb the brush roller 14 on the other hand.

For the safety of the surface cleaning system, a temperature indication module (not shown in the figure) and a temperature detection component can also be provided on the base station 20. The temperature detection component is connected to the heating wall structure 8. The temperature indication module can inform the user the temperature detected by the temperature detection component, that is, to indicate the temperature of the heating wall structure 8 to the user. This ensures that the user touches the base station 20 within a safe temperature range. The temperature indication module can comprise a temperature indicator light, a temperature indicator bar, or a temperature display panel. Preferably it is convenient for the user to observe. Of course, the temperature indication module might be omitted in some embodiments.

In some embodiments, a status display light (not shown in the figure) is further provided on the base station 20, and the control unit 30 is in communication with the status display light. The control unit controls the status display light to be in a lit state during the operation of the heating wall structure 8, which reminds the user that the heating wall structure 8 is currently in operation.

The tray portion 51 comprises a bottom plate 511 facing the ground, and the bottom plate 511 is at least partially made of heat insulating material. The bottom plate 511 made of the heat insulating material reduces heat loss and effectively prevents heat transfer from the heating wall structure 8 to the ground, thereby avoiding damage to the ground.

As shown in FIG.6, the dimension of the heating wall structure 8 is configured to satisfy the following requirements: when the surface cleaning device 10 is docked at the base station 20, the heating wall structure 8 circumferentially encloses the periphery of the exposed portion 141 (the shaded portion of the brush roller in the figure) and contacts the exposed portion 141 of the brush roller 14. The enclosure here may be to enclose the entire exposed portion 141 or to enclose part of the exposed portion 141.

The heating wall structure 8 in this embodiment can heat the brush roller 14, the water flowing into the recess 6, and the air flowing through the recess 6. The heating wall structure 8 is controlled to start heating when the surface cleaning device 10 is docked at the base station 20. The heating wall structure 8 circumferentially encloses the outer periphery of the exposed portion 141 of the brush roller 14 when the surface cleaning device 10 is docked at the base station 20, and the upper surface 801 of the heating wall structure 8 contacts the brush roller 14. These structures enable the heat generated by the heating wall structure 8 to be quickly transferred to the brush roller 14, and the enclosure structure also limits the escape of the heat generated by the heating wall structure 8.

Referring to FIG.7, the control unit 30 of the surface cleaning system of the present application comprises two parts, one part is located in the surface cleaning device 10, namely, a first controller 301 located in the surface cleaning device 10. The other part is located in the base station 20, namely, a second controller 302 located in the base station 20. These two controllers achieve the control of various controllable components of the surface cleaning system. In this embodiment, the first controller 301 in the surface cleaning device 10 controls whether the suction motor 42, the pump 33 and the brush roller motor 16 take part in the operation. The second controller 302 in the base station 20 controls whether the heating element 82 takes part in the operation, and controls the operation of the temperature indication module (if any) and the status display light (if any). In other embodiments, the control unit of the surface cleaning system can also be fully integrated in the surface cleaning device. According to this solution, the heating element in the base station will also be controlled by the control unit in the surface cleaning device to operate. Thus, after the surface cleaning device and the base station are docked, a path for instruction transmission must be constructed.

When the surface cleaning device 10 is docked at the base station 20, the brush roller 14 is located at the recess 6. Thereby the surface cleaning system can perform self-cleaning, sterilizing and drying process for the surface cleaning device under the control of the control unit 30, specifically including: a self-cleaning process for cleaning the brush roller with water, a sterilizing process for sterilizing the brush roller with steam, and a drying process for drying the brush roller. Of course, the self-cleaning process, sterilizing process and drying process are likewise applied to the recovery flow path of the surface cleaning device.

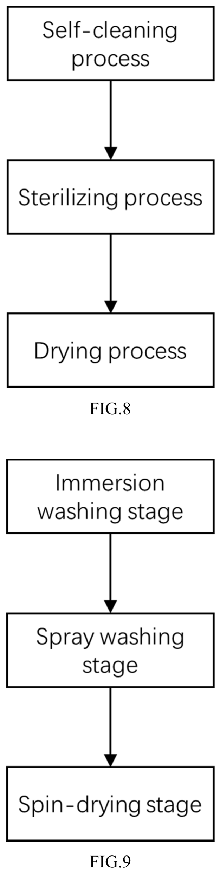

As shown in FIG.8, the self-cleaning process, sterilizing process and drying process in this embodiment are configured to be performed in sequence. In this way, the user only needs to interact with the surface cleaning system once, and the program containing these processes will be triggered, so that the self-cleaning process, sterilizing process and drying process are performed in sequence. The interaction between the user and the surface cleaning system can be achieved by triggering a program switch arranged on the surface cleaning device or the base station. In other embodiments, the interaction between the user and the surface cleaning system can also be omitted, and instead a monitoring device is used to monitor constantly whether the surface cleaning device is docked at the base station. When it is detected that the surface cleaning device is docked at the base station, the above-mentioned self-cleaning process, sterilizing process and drying process are automatically started. The start time of the self-cleaning process, sterilizing process and drying process depends on whether the respective processing conditions are met. For example, regarding the sterilizing process, the conditions for starting the process comprise whether the self-cleaning process has been completed, etc.

In other feasible embodiments, the sterilizing process or the drying process may also be configured as a standalone program for the user to select and execute.

As shown in FIG.9, the self-cleaning process of this embodiment comprises the following stages performed in sequence:
Immersion cleaning stage: firstly, water is continuously supplied to the brush roller 14 from the water tank 31 for a period of time, and the suction motor 42 is not started at this time. In this step, water not absorbed by the brush roller 14 will flow into the recess 6, and water supply will continue until a sufficient amount of water is collected in the recess 6. At this time, the heating element 82 in the recess 6 generates heat under the control of the second controller 302, and the heat of the heating element 82 is transferred to the heat-con6ducting part 81, and the brush roller 14 is heated, and the water in the recess 6 is continuously heated to become hot water with higher temperature. In this process, the brush roller 14 may be driven to rotate or may not rotate. Secondly, the water supply is stopped, and the first controller 301 controls the brush roller motor 16 to drive the brush roller 14 to rotate continuously for a period of time. In this step, the brush roller 14 is at least partially immersed in the hot water in the recess 6, and the continuous rotation of the brush roller 14 can achieve immersion cleaning of the brush roller 14. Thirdly, the first controller 301 controls the suction motor 42 to turn on, and keeps the brush roller 14 rotating continuously, so as to transfer the water after immersion cleaning from the recess 6 to the dirty liquid recovery tank 41 via the recovery flow path 43.

Spray cleaning stage: water is supplied to the brush roller 14 again from the water tank 31, and the first controller 301 controls the suction motor 42 to turn on and controls the brush roller motor 16 to drive the brush roller 14 to rotate. At this time, the heating element 82 continues to generate heat under the control of the second controller 302, and the heated heat-conducting part 81 continues to heat the brush roller 14. Liquid falling from the brush roller 14 will be transferred to the dirty liquid recovery tank 41 via the recovery flow path 43. In this step, "new" water is continuously distributed to the brush roller 14, and at the same time, the "old" water falling from the brush roller 14 is transferred to the dirty liquid recovery tank 41 via the recovery flow path 43. The continuous rotation of the brush roller 14 can achieve spray cleaning of the brush roller 14.

Spin-drying stage: water supply is stopped, and the suction motor 42 is turned on and the brush roller 14 is driven to rotate continuously for a period of time to spin-dry the water on the brush roller 14 as much as possible. During this stage, the heating wall structure 8 at the recess 6 continues to heat.

After the above-mentioned stages are completed in sequence, the entire self-cleaning process is completed.

In the present application, regarding the self-cleaning process of the brush roller, since the heating element generates heat during the entire process, the cleaning process of the brush roller is carried out in a heated environment. The heated environment not only heats the brush roller, but also heats the water used to clean the brush roller, which is greatly beneficial to the cleaning of the brush roller. Compared with cleaning the brush roller in a normal temperature environment with room-temperature water, this solution cleans the brush roller more thoroughly.

In the above-mentioned immersion cleaning stage, the amount of water collected in the recess 6 can be configured according to the volume of the recess and the water absorption rate of the cleaning element. And in this immersion cleaning stage, the brush roller 14 can be partially immersed in the hot water in the recess 6, that is, in a semi-immersed status.

During the self-cleaning process, the first controller 301 controls the brush roller motor 16 to drive the brush roller 14 to rotate at a first rotation speed, which can be equal to the rotation speed of the brush roller when the surface cleaning device 10 performs surface cleaning operation, or it can be greater than or less than the rotation speed of the brush roller when the surface cleaning device 10 performs surface cleaning operation. It can be determined according to the effect of the self-cleaning process of the brush roller.

In other embodiments, during the self-cleaning process, the immersion cleaning stage and the spray cleaning stage of the brush roller can be performed selectively, such as only performing the spray cleaning stage once. In addition, in some feasible embodiments, the water used in the self-cleaning process can also come from outside the water tank, such as from a water storage container on the base station, or even from a tap water pipe connected to the base station. The purpose of the self-cleaning process is to clean the brush roller, and any solution that is convenient for cleaning the brush roller can be applied here.

The sterilizing process in this embodiment is performed after the self-cleaning process is completed. The purpose of the sterilizing process is to use steam to sterilize the cleaned brush roller. During the sterilizing process, the second controller 302 controls the heating wall structure 8 to generate heat. The steam used will be generated by the heating wall structure 8 heating the water in the recess 6 and/or heating the cleaning element 1402 soaked in water. The water can be the water supplied to the brush roller 14 from the water tank 31, or it can come from other places. In the sterilizing process, in order to instantaneously generate a large amount of steam and increase the steam temperature, the amount of water supplied to the brush roller during this process should be increased relative to the amount of water in the self-cleaning process. The water in the recess 6 and/or the water absorbed by the cleaning element 1402 can be injected at one time or in stages.

During the sterilizing process, the suction motor 42 is not started, and the first controller 301 controls the brush roller motor 16 to drive the brush roller 14 to rotate at a second rotation speed, which is preferably less than the first rotation speed, that is, during the sterilizing process, the brush roller 14 maintains a relatively low-speed rotation in a steam-filled environment.

In other embodiments, the sterilizing process may also be cancelled, or combined with the self-cleaning process and performed simultaneously.

The drying process in this embodiment is performed after the self-cleaning process or the sterilizing process (if any) is completed. The purpose of the drying process is to further dry the brush roller to prevent the brush roller from generating odor or mildew later. During the drying process, the suction motor 42 is started under the control of the first controller 301, and the brush roller motor 16 is also started under the control of the first controller 301, and the heating element 82 is heated under the control of the second controller 302, so that the heat-conducting part 81 is heated. At this time, the heat-conducting part 81 can directly heat the brush roller 14. The brush roller 14 will rotate at a constant speed under the drive of the brush roller motor 16. In this way, under the action of the heating wall structure 8 and the suction motor 42, the air located at the recess 6 will be heated into hot air by the heat-conducting part 81 and/or the brush roller 14, and the hot air will further flow along the recovery flow path 43 from the suction nozzle 19 toward the dirty liquid recovery tank 41. Under the heating effect of the heating wall structure 8 and the convection effect of the hot air, the moisture on the brush roller 14 will evaporate quickly and be quickly taken away by the flowing hot air, thereby achieving the effect of quickly drying the brush roller. At the same time, when the hot air flows through the recovery flow path 43, it will also quickly take away the residual moisture in the recovery flow path 43.

During the drying process, in order to reasonably utilize electrical energy, the heating element 82 may stop heating in advance before the suction motor 42 and the brush roller motor 16 are turned off, thus the residual heat of the heating element 82 and the heat-conducting part 81 may be utilized to completely dry the brush roller 14.

During the drying process, the brush roller motor 16 drives the brush roller 14 to rotate at a third rotation speed under the control of the first controller 301, and the third rotation speed is preferably equal to the rotation speed of the brush roller of the surface cleaning device 10 when performing the surface cleaning operation, that is, during the sterilizing process, the brush roller 14 also rotates at a relatively high speed, which can be hundreds of revolutions per minute or even thousands of revolutions per minute. In fact, the greater the rotation speed of the brush roller, the easier it is to dry the brush roller. However, the faster the rotation speed, the higher the voltage of the brush roller motor, which will affect the service life of the brush roller motor. Therefore, when configuring the rotation speed of the brush roller during the drying process, it is necessary to balance the drying speed and the service life of the brush roller motor.

During the drying process of the present application, the heat for the drying process comes from the heating element 82 of the heating wall structure 8, and the air flow power comes from the suction motor 42. Compared with the traditional method of drying the brush roller by hot air generated by a fan or a blower combined with a heating component, this solution can transfer more heat to the brush roller 14 per unit time, and the air flow speed can also be faster, so that the heat exchange at the recess 6 is significantly more intense. The heat radiation and heat conduction of the heat-conducting part 81 of the heating wall structure 8 to the brush roller 14, superimposed on the convection heat exchange effect of the air, accelerate the drying of the brush roller.

The above embodiments are only for illustrating the technical concept and features of the present application, and their purpose is to enable people familiar with the technology to understand the content of the present application and implement it accordingly. They should not be used to limit the protection scope of the present application. Any equivalent changes or modifications made according to the spirit of the present application should be comprised in the protection scope of the present application.

## Claims

1. A surface cleaning system, **characterized by** comprising:
a surface cleaning device, comprising a suction nozzle, a brush roller, a brush roller motor for driving the brush roller to rotate, a dirty liquid recovery tank and a suction motor, wherein the brush roller comprises a roller body and a cleaning element which covers an outer peripheral surface of the roller body and is capable of being wetted by liquid, and the brush roller has an exposed portion exposed outward;
a base station, configured to allow the surface cleaning device to dock, wherein the base station comprises at least one heating wall structure having a controllable heating element, and the at least one heating wall structure is configured to circumferentially cover at least a portion of an outer periphery of the exposed portion while the surface cleaning device is docked at the base station; and
a control unit, wherein the control unit is at least partially arranged at the surface cleaning device, and the control unit is configured to perform a self-cleaning process of cleaning the brush roller with water, and a drying process of drying the brush roller while the surface cleaning device is docked at the base station; wherein during execution of the drying process, the control unit is configured to control the suction motor and the brush roller motor respectively to be in continuous operation and to control the heating element to generate heat for at least a period of time.

2. The system according to claim 1, **characterized in that**
the at least one heating wall structure has an upper surface facing the brush roller, and the heating element is configured so that a temperature of the upper surface is greater than or equal to 50°C during the drying process and when the heating element is in a heating period.

3. The surface cleaning system according to claim 2, **characterized in that**
the upper surface is arc-shaped.

4. The surface cleaning system according to claim 1, **characterized in that**
the at least one heating wall structure is configured to contact the exposed portion while the surface cleaning device is docked at the base station.

5. The system according to claim 1, **characterized in that**
the base station comprises a recess capable of receiving at least a portion of the brush roller while the surface cleaning device is docked at the base station, and the at least one heating wall structure comprises at least a portion of a wall of the recess.

6. The system according to claim 5, **characterized in that**
the recess has a front wall and a bottom wall, and the at least one heating wall structure comprises at least a portion of the front wall and at least a portion of the bottom wall.

7. The system according to claim 1, **characterized in that**
the at least one heating wall structure comprises a heat-conducting part, and the heating element is embedded in the heat-conducting part.

8. The system according to claim 1, **characterized in that**
the at least one heating wall structure circumferentially covers more than fifty percent of a surface area of the exposed portion while the surface cleaning device is docked at the base station.

9. The system according to claim 1, **characterized in that**
the self-cleaning process is configured to be performed before the drying process, and the control unit is configured to control the heating element to generate heat during the self-cleaning process.

10. The system according to claim 1, **characterized in that**
the surface cleaning device also comprises: a water tank, a liquid distributor facing the brush roller, and a pump for conveying liquid from the water tank to the liquid distributor; wherein the water used during the self-cleaning process comes from the water tank.

11. The surface cleaning system according to claim 1, **characterized in that**
the control unit comprises a first controller located at the surface cleaning device, and a second controller located at the base station, and the first controller is in communication with the second controller while the surface cleaning device is docked at the base station.

12. The system according to claim 1, **characterized in that**
the control unit is configured to perform a sterilizing process of the brush roller using steam while the surface cleaning device is docked at the base station, and the sterilizing process is configured to be performed between the self-cleaning process and the drying process; wherein the control unit is configured to control the heating element to generate heat to heat water in the recess and/or the cleaning element soaked in water to generate the steam during the sterilizing process, and the control unit is configured to control the brush roller motor to drive the brush roller to rotate during the sterilizing process.

13. The system according to claim 12, **characterized in that**
during the sterilizing process, the control unit controls the brush roller motor to drive the brush roller to rotate at a speed lower than a rotation speed of the brush roller when the surface cleaning device performs a surface cleaning operation.

14. The system according to claim 1, **characterized in that**
during the drying process, the control unit controls the heating element to stop heating in advance before the drying process ends.

15. A method for drying a brush roller, applied to a surface cleaning device, **characterized in that** the method comprises:
docking the surface cleaning device to a base station provided with at least one heating wall structure, wherein the at least one heating wall structure comprises a controllable heating element and the at least one heating wall structure is configured to circumferentially cover at least a portion of a periphery of the brush roller during a period when the surface cleaning device is docked at the base station; and
controlling the heating element to generate heat so as to heat the brush roller via the at least one heating wall structure, and starting a brush roller motor of the surface cleaning device and a suction motor of the surface cleaning device to both participate in operation.

16. The method according to claim 15, **characterized in that**
the at least one heating wall structure contacts the brush roller while enclosing at least a portion of a periphery of the brush roller.

17. The method according to claim 15, **characterized in that**
the method further comprises: controlling the heating element to stop heating in advance before shutting down the brush roller motor and the suction motor.

18. A method for cleaning a brush roller, applied to a surface cleaning device, **characterized in that** the method comprises:
docking the surface cleaning device to a base station provided with at least one heating wall structure to clean the brush roller; wherein the at least one heating wall structure comprises a controllable heating element and the at least one heating wall structure is configured to circumferentially cover at least a portion of a periphery of the brush roller while the surface cleaning device is docked at the base station; and
controlling the heating element to generate heat so that the brush roller is cleaned in a heated environment.

19. The method according to claim 18, **characterized in that**
the method comprises: applying water required for cleaning the brush roller to the brush roller.

20. The method according to claim 18, **characterized in that**
the method comprises: transferring used water that has been used for cleaning the brush roller from the brush roller.

21. The method according to claim 20, **characterized in that**
the surface cleaning device is also provided with a suction nozzle, a dirty liquid recovery tank and a suction motor, and the method comprises: transferring the used water that has been used for cleaning the brush roller from the brush roller to the dirty liquid recovery tank utilizing the suction motor.

22. The method according to claim 18, **characterized in that**
the heating wall structure contacts the brush roller while circumferentially covering at least a portion of the periphery of the brush roller.

23. A method for sterilizing a brush roller, applied to a surface cleaning device, **characterized in that** the method comprises:
docking the surface cleaning device to a base station provided with at least one heating wall structure, wherein the at least one heating wall structure comprises a controllable heating element and the at least one heating wall structure is configured to circumferentially cover at least a portion of a periphery of the brush roller during a period when the surface cleaning device is docked at the base station;
controlling the heating element to generate heat so as to convert water located at the at least one heating wall structure and/or water absorbed by the brush roller into steam; and
controlling a brush roller motor of the surface cleaning device to drive the brush roller to rotate in a steam-filled environment.

24. The method according to claim 23, **characterized in that** the method further comprises:
controlling the brush roller motor to drive the brush roller to rotate in a steam-filled environment at a rotation speed lower than the rotation speed of the brush roller when the surface cleaning device performs a surface cleaning operation.
